# EUROPEAN PATENT APPLICATION

(11) **EP 2 047 849 A1**
(43) Date of publication of application: **15.04.2009**
(21) Application number: 07019619.1
(22) Date of filing: 08.10.2007
(51) Int. Cl.: A61K 31/407, A61P 35/00, A61P 35/02

(54) **Use of indolocarbazole imides as protein kinase inhibitors for the treatment of hematologic and solid tumors**

(71) Applicant: KTB Tumorforschungsgesellschaft mbH, 79106 Freiburg (DE)
(72) Inventor: The designation of the inventor has not yet been filed
(74) Representative: Miller, Andreas

(57) **Abstract**

The invention comprises the inhibition of selected protein kinases for the treatment of hematologic and solid tumors in humans by selected indolocarbazole imides.

## Description

### EP-Patent 0 642 513 describes indolocarbazole imides of the general Formula (I)

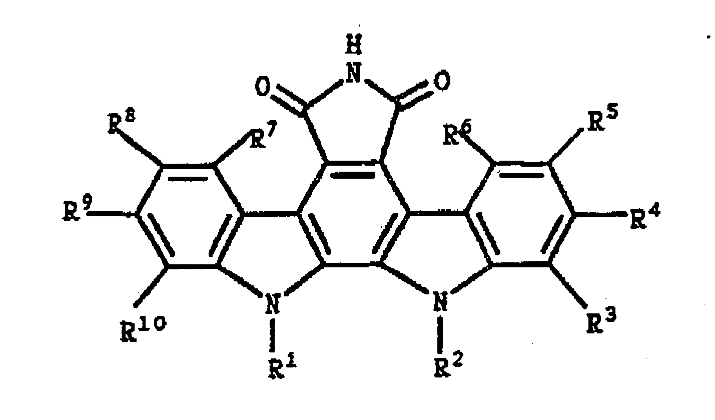

in which R¹ and R² are the same or different and, in each case, are a hydrogen atom or a methyl, ethyl, cyanomethyl, 2-cyanoethyl, 3-cyanopropyl, 3-cyano-2-propyl, 2-azidoethyl, 3-azidopropyl, 2,3-dihydroxypropyl, 3-chloro-2-hydroxy-1-propyl, 3-amino-propyl, 3-dimethylaminopropyl, 3-trimethylammoniopropyl, 4-aminobutyl, 5-aminopentyl, epoxymethyl, 3-methylaminopropyl, 3-ethylaminopropyl, 3-isopropylaminopropyl, 3-diisopropylaminopropyl, 3-methylamino-2 -hydroxy-1-propyl, 3 -ethylamino-2 -hydroxy-1-propyl, 3-isopropylamino-2-hydroxy-1-propyl, 3-dimethylamino-2-hydroxy-1-propyl, 3-diethylamino-2-hydroxy-1-propyl, 3-diisopropylamino-2-hydroxy-1-propyl, 3-pyrrolidino-2-hydroxy-1-propyl or the radical - (CH₂)₂CONH₂ and R³ to R¹⁰, independently of one another, each stand for hydrogen, methyl, methoxy, n-propoxy, chlorine, bromine, nitro, hydroxyl or amino or two neighbouring radicals together stand for a methylenedioxy radical as well as of the pharmacologically acceptable salts thereof and their use for the preparation of pharmaceutical compositions. Some compounds by novelty reasons had been excluded by proviso.

Basic compounds of general formula (I) which have a basic centre on at least one of the radicals are, for the purpose of purification and for galenical reasons, preferably converted into crystalline, pharmacologically acceptable salts. The salts are obtained in the usual manner by neutralisation of the bases with appropriate inorganic or organic acids. As acids, there can be used, for example, hydrochloric acid, sulphuric acid, phosphoric acid, hydrobromic acid, acetic acid, tartaric acid, lactic acid, citric acid, malic acid, salicylic acid, ascorbic acid, malonic acid, fumaric acid, oxalic acid or succinic acid. The acid addition salts are, as a rule, obtained in known manner by mixing the free base or a solution thereof with the appropriate acid or a solution thereof in an organic solvent, for example a lower alcohol, such as methanol, ethanol or propan-2-ol, or a lower ketone, such as acetone or butan-2-one, or an ether, such as diethyl ether, diisopropyl ether, tetrahydrofuran or dioxane. Compounds of general formula (I) which have a chiral centre in the radicals R³ to R¹⁰ can be used as stereoisomeric mixtures or in the form of the enantiomers. The enantiomers can be obtained by means of the processes normally employed for the optical separation of stereoisomers. The use of such salts and isomeric forms is comprised by the present invention.

At the filing date of EP-Patent 0 642 513 the compounds of formula (1) had been provided as pharmaceutically active compounds for a quite broad spectrum of diseases, namely the treatment and/or the prevention of cancer, virus diseases (for example HIV infections), heart and blood vessel diseases (for example high blood pressure, thromboses, heart rhythm disturbances and atherosclerosis), bronchopulmonary diseases, degenerative diseases of the central nervous system (for example Aizheimer's disease), inflammatory diseases (for example rheumatism and arthritis), diseases of the immune system (for example allergies), as well as psoriasis and for use as immune suppressives. Depending upon the substitution, compounds of general formula (I) are described as potent Inhibitors of protein kinase C or of myosin light-chain kinase.

In EP-Patent 0 642 513 compounds of general formula (I) in which R¹ and R² have the above-given meanings but at least one of the radicals R¹ and R² is other than a hydrogen atom and R³ to R¹⁰ have the above-given meanings are preferred. Despite the fact, that the above compounds already 10 years ago had been broadly investigated the above spectrum of activity was never better specified.

Regulation of different cellular functions such as cell division is achieved by a signal transduction network of which protein kinases are key players. The human genome project revealed that there are 518 different human protein kinase genes. In various diseases genetically manifested deregulated of protein kinases leads to disturbances of the cellular signal transduction network resulting in a loss of controll of e.g, cell proliferation that contributes to the development and maintenance of diseases like cancer. Cancer is a group of often life-threatening diseases with a high medical need sharing uncontrolled cell proliferation as one common feature. In different types of cancer deregulation of cell proliferation is caused by different genetic alteration affecting different genes. In many cases these genes encode for protein kinases and the alterations result in a pathological increase of their activity. Due to the structural similarity of protein kinases low molecular inhibitors often inhibit not only one protein kinase, but a selected group of them. The invention is based on the fact that it was found that tumors frequently depend on the increased activity of different protein kinases that regulate different biological pathways Inhibitors that are able to inhibit two or more tumor growth promoting protein kinases could improve the effectiveness of tumor therapy. Depending on the type of cancer deregulation of different protein kinases occurs. For example 95% of all patients suffering from chronic myeloid leukemia (CML) a chromosomal translocation of the gene encoding the protein kinase Abl is found (Philadelphia-Chromosome). This translocation results in an increased activity of the Abl protein kinase causing enhanced cell proliferation. Low molecular weight inhibitors of Abl such as Gleevec^{®} (Novartis) have been developed and were approved and are successfully used for the treatment of CML. Additionally Gleevec^{®} also inhibits protein kinase c-Kit that is often found deregulated in gastrointestinal stromal tumors (GIST) and based on this activity Gleevec^{®} is also approved for treatment of c-Kit positive GIST. Initially, simultaneous inhibition of several protein kinases was thought to be of disadvantage due to potentially unacceptable side effects. However, protein kinase inhibitors have been approved recently that do inhibit a significant higher number of protein kinases e,g. Sutent^{®} (Pfizer) and Sorafenib^{®} (Bayer). Sutent^{®} for example inhibits, among others, VEGF-R, c-Kit, FLT3 and FGF-R kinases. Both compounds are approved for the treatment of GIST and advanced renal cell carcinoma (RCC) demonstrating that simultaneous inhibition of different protein kinases is not counter-indicative. In contrast, inhibition of multiple protein kinases is nowadays thought to be potentially more effective in the treatment of cancer, since tumor growth is inhibited by blocking several tumor growth promoting mechanisms simultaneously. It has been shown that Sutent^{®} and Sorafenib^{®} act on different tumor promoting mechanisms (proliferation and tumor induced blood vessel formation) and it is thought that this might be one reason for their effectiveness in the treatment of cancer.

The present invention comprises a group of low molecular weight compounds that act as multi-protein kinase inhibitors being surprisingly effective and potent to the protein kinases FLT3, FLT3 wild type mutant as FLT D835 or FLT3-ITD, PDGF-Rα, PDGF-Rβ, CHK2, RSK family members RSK1-4, FAK and MAP4K4, Aurora B, PLK4 and PRKG2. Compounds of formula (1) thus can be selectively used for the treatment of different tumor types e.g. acute myeloid leukemia (AML) showing increased activity of FLT3 kinases and different solid tumors e,g, lung, breast or colon tumors that show deregulated PDGF-R, RSK, MAP4K4, or CHK2 kinases.

Acute myeloid leukaemia (AML) is one of the most frequently occurring forms of leukemia with a high medical need. The majority of the AML-patients relapse after initial chemo-therapy, and the 5-year survival rate is about 30-40%. in at least 30% of all AML-patients activity of the protein kinase FLT3 is increased due to different genetic alterations of the FLT3 gene (e.g. internal tandem repeat mutant; ITD or point mutations such as D835Y, as mentioned above). Increased activity of FLT3 results in activation of the Ras/MAPK-signalling pathway leading to increased proliferation, enhanced survival and reduced differentiation of the tumor cells. Inhibition of FLT3 by the low molecular weight inhibitors of the present invention blocks cell proliferation and induce programmed cell death in AML-cells. A second protein kinase that has been found to be frequently overexpressed in AML-cells is the PDGF-Receptor (PDGF-R) which upon activation by its ligand PDGF enhances proliferation of AML-cells. Hence, simultaneous inhibition of FLT3 and PDGF-R in AML cells can be expected to lead to a more efficient inhibition of the proliferation of AML cells and to a more efficient treatment of AML. Tumors of solid tissues represent the majority of human cancers and the many different tumor types classified the histological parameters (e,g, lung, breast, colon tumors etc) show an immense variety in the identity and combination of deregulated protein kinases that they express. Growth of many solid tumors is often supported by the PDGF-R that acts via two different tumor growth promoting mechanisms. Overexpression of the receptor (and often simultaneous overexpression of the activating ligand PDGF) in the tumor cells stimulates the growth of the tumor cell itself (autokrine mechanism), This is frequently observed in glioblastomas and sarcomas. A second mechanism is often observed in epithelial tumors (such as colorectal, lung, and breast tumors) that do not express the PDGF-Receptor, but the activating ligand (paracrine mechanism). The tumor cell derived ligand subsequently activates the PDGF-receptor of normal cells like fibroblasts, smooth muscle cells. pericytes and endothelial cells that surround the tumor (tumor stroma). As a consequence these stroma cells form a tumor growth promoting microenvironment. CHK2 is a protein kinase that regulates a cell division cycle checkpoint. In normal cells CHK2 is activated after occurrence of DNA-damage either by inducing a stop in cell division and DNA-repair or by initiating apoptosis (cell death) to prevent the accumulation of genetically altered cells that might result in a transformation of normal cells into tumor cells. Accumulation of mutations and chromosomal alteration is a common feature of tumor cells, but tumor cells are protected by apoptosis via the CHK2 checkpoint because this pathway is frequently inactivated in tumor cells. The mechanisms by which this checkpoint is altered in tumor cells are divers. Subsets of different types of tumors have lost CHK2 expression (e.g. breast, lung, colon cancer). Others show increased activity (e.g. in primary breast and colon tumors) of this kinase, many of which have inactivated p53, one of the substrates of CHK2. In this case CHK2 is probably partially inactivated in that way that the activated CHK2 pathway induces DNA-repair without being able to induce cell death. Two strategies for the use of inhibitors of CHK2 depending on the CHK2 status of the tumor cells can be explored. First, inhibition of CHK2 in tumor cells that depend on overexpression of CHK2 to prevent apoptosis due to accumulation of genetic alteration could restore the apoptotic response inducing cell death in this type of tumor cells. Secondly, in tumors that have lost CHK2 expression efficiency of treatment with unspecific chemotherapeutics could be improved, because inhibition of CHK2 in normal healthy cells could prevent induction of cell death by chemotherapeutics by preventing CHK2 induced apoptosis in healthy cells thus reducing the undesired side effect of common chemotherapeutics which represent often a limitation in the use of this type of drugs, About 50% of human breast tumors show overexpression of the deregulating protein kinases of the RSK family. RSK is for example activated by EGF via the EGF-R, a proven target in cancer. Furthermore, inhibition of RSK in a human breast cancer cell line results in reduction of cell proliferation demonstrating that inhibition of RSK protein kinases itself represents a potential therapeutic approach.

It is a subject of the present invention to improve especially the therapy of those types of cancer which are promoted by the deregulating protein kinases FLT3 wild type, FLT D835 mutant, PDGF-Rα, PDGF-Rβ, CHK2, FAK, MAP4K4, Aurora B, PLK4, PRKG2,and RSK1-4 in humans by using the compounds of formula (I) as special inhibitors of said kinases. It has been found that the kinases FLT3 wild type, as e.g. FLT D835 mutant or FLT3-ITD, and PDGF-Rα, PDGF-Rβ are preferably promoting hematologic tumors especially myelogenous leukemia (AML) while CHK2, FAK, MAP4K4, Aurora B, PLK4, PRKG2,and RSK1-4 are more deregulated in solid tumors, especially lung, breast, kidney, prostate, pancreas and colon tumors. The inventors' findings surprisingly now allow the especially targeted treatment of extremely perilous tumors as e.g.myelogenous leukemia (AML) and tumors of the pancreas. It was hitherto unkown and could not be expected by a person skilled in the art that the compounds of the present invention allow such a specific and effective inhibition of the selected kinases promoting a series of hardly curable malign tumors.

Preferred compounds are those of formula (I) wherein R¹ and R² have the above-given meanings but at least one of the radicals R¹ or R² is other than a hydrogen atom and R³ to R¹⁰ have the above-given meanings.

Furthermore preferred are compounds of general formula (I) which have a chiral center in the radicals R¹ to R¹⁰ and are used as stereo-isomers or in the form of enantiomers.

Especially preferred are compounds of formula (I), wherein R¹ and/or R² is hydrogen, C1-C5 alkyl, amino-C1-C5 alkyl, mono- or bis- hydroxylated amino- C1-C5 alkyl, C1-C3 alkylamino- C1-C5 alkyl, mono or bis hydroxylated C1-C3 alkylamino-C1-C5 alkyl, carbonamido- C1-C4 alkyl, cyano- C1-C3 alkyl and R³ to R¹⁰ hydrogen and/or hydroxy or C1-C3-alkoxy-groups.

More preferred are compounds of formula (I), wherein R¹ and/or R² is hydrogen, methyl, dimethyl-aminoethyl, dimethyl-aminopropyl, 2-carbonamido-ethyl, 2-hydroxy-3-dimethylamino-propyl or 2-cyano-ethyl and the remaining radicals are as described above, preferably hydroxy and/or methoxy.

The most preferred compounds are:
13-(2-Cyanoethyl)-6,7,12,13-tetrahydro-3-methoxy-12-methyl-5,7 dioxo-5H-indolo[ 2,3-a] pyrrolo [3,4-c]-carbazole (PQ 014 = Ex.1);
12-(3-Dimethylaminopropyl)-6,7,12,13-tetrahydro-3,9-dihydroxy-12-methyl-5,7 dioxo-5H-indolo[2,3-a] pyrrolo [3,4-c]-carbazole (PQ 012 = Ex.2c);
6,7,12,13-Tetrahydro-12-methyl-5,7 dioxo-5H-indolo [2,3-a] pyrrolo [3,4-c]-carbazole (PQ 042 = Ex. 7c);
6,7,12,13-Tetrahydro-3,9-dimethoxy-12-methyl-5,7 dioxo-5H-indolo [2,3-a] pyrrolo [3,4-c]-carbazole (PQ 013 = Ex. 7b);
12-(2-Carbamidoethyl)-6,7,12,13-tetrahydro-5,1 dioxo-5H-indolo[2,3-a] pyrrolo [3,4-c]-carbazole (PQ 041 = Ex.5);
(+-)-12-(3-Dimethylamino-2-hydroxy-1-propyl)-6,7,12,13-tetrahydro-3,9-dihydroxy-13-methyl -5,1 dioxo-5H-indolo[2,3-a] pyrrolo [3,4-c]-carbazole (PQ 016 = Ex.10);

The Example numbers (Ex.) refer to the Examples of EP-Patent 0 642 513.

The most preferred compound (PQ 013) for the use according to the present invention is the compound of formula (I), wherein R¹, R³, R⁴, R⁶, R⁷, R⁹ and R¹⁰ are all hydrogen, R² is methyl and R⁵ and R⁸ are both methoxy.

The compounds of general formula (I) according to the present invention can be administered orally or parenterally in liquid or solid form. As injection medium, water is preferably used which contains the additives usual in the case of injection Solutions, such as stabilising agents, solubilising agents or buffers. Additives of this kind include, for example, tartrate and citrate buffers, ethanol, complex formers (such as ethylenediamine-tetraacetic acid and the nontoxic salts thereof), as well as high molecular weight polymers (such as liquid polyethylene oxide) for viscosity regulation. Solid carrier materials include, for example, starch, lactose, mannitol, methyl cellulose, talc, highly dispersed silicic acids, high molecular weight fatty acids (such as stearic acid), gelatin, agar-agar, calcium phosphate, magnesium stearate, animal and vegetable fats and solid high molecular weight polymers (such as polyethylene glycol). Compositions suitable for oral administration can, if desired, contain additional flavouring and/or sweetening materials. The compounds can be administered enterally or parenterally in the particularly appropriate formulation in doses of from 1 to 500 mg/kg and preferably of from 1 to 50 mg/kg.
The enantiomers can be obtained according to EP-Patent 0 642 513 by means of the processes which are usually employed for the optical separation of stereoisomers.

### Material and Methods

### In vitro protein kinase actively assay

Inhibition of protein kinases by compounds was measured in proprietary protein kinase activity assay (³³PanQinase^{®} Activity Assay) using recombinant purified protein kinases. All protein kinases were expressed in Sf9 insect cells as recombinant GST-fusion proteins or His-tagged proteins by means of the baculovirus expression system. All kinases were produced from human cDNAs. Kinases were purified by affinity chromatography using either GSH-agarose (Sigma) or Ni-NTH-agarose (Qiagen). The purity of the protein kinases was examined by SDS-PAGE/Coomassie staining. Activity was measured by determination of the incorporation of radio labelled phosphate into a substrate. The reaction cocktails for all enzymes contained 60 mM HEPES-NaOH, 10 mM Tris/HCl, pH 7.5, 20 mM NaCl, 3 mM MgCl₂, 3 mM MnCl₂, 3 µM Na-orthovanadate, 2 mM DTT, 4% glycerol, 1 µM [γ-³³P]-ATP (approx. 6 x 10⁵ cpm per well), protein kinase , and substrate.

**Table 1: Protein kinases inhibited with high potency in vitro by compound PQ-012, PQ-013, PQ-014, PQ-016, PQ-041, PQ-042.**

| **Kinase** | **PQ-012** | **PQ-013** | **PQ-014** | **PQ-016** | **PQ-041** | **PQ-042** |
|---|---|---|---|---|---|---|
| Aurora B | n.t. | 2.1E-07 | n.t. | n.t. | n.t. | n.t. |
| CHK2 | 2.3E-08 | 4.0E-08 | 2.1E-06 | 2.2E-05 | 7.4E-07 | >1E-05 |
| FAK | 1.4E-08 | 3.6E-05 | 1.2E-06 | 1.0E-06 | 2.4E-06 | >1E-05 |
| FLT3 wt | <3E-09 | 4.6E-09 | 4.6E-09 | 1.1 E-07 | 5.6E-09 | 3.8E-07 |
| FLT3 D835Y | <3E-09 | <3E-09 | 3.5E-09 | 6.4E-08 | 3.9E-09 | 6.8E-08 |
| MAP4K4 | n.t. | 3.5E-08 | n.t. | n.t. | n.t. | n.t. |
| PDGFR-alpha | <3E-09 | 1.0E-08 | 1.1E-07 | 2.0E-06 | 1,3E-07 | >1E-05 |
| PDGFR-beta | <3E-09 | 1.9E-08 | 1,3E-07 | 7,0E-07 | 1,5E-07 | >1E-05 |
| PRKG2 | n.t. | 6.7E-08 | n.t. | n.t. | n.t. | n.t. |
| PLK4 | n.t. | 1.8E-07 | n.t. | n.t. | n.t. | n.t. |
| RSK1 | n.t. | 2.30E-08 | n.t. | n.t. | n.t. | n.t. |
| RSK2 | n.t. | 4.30E-07 | n.t. | n.t. | n.t. | n.t |
| RSK3 | n.t. | 1.70E-07 | n.t. | n.t. | n.t. | n.t. |
| RSK4 | n.t. | 7.80E-08 | n.t. | n.t. | n.t | n.t. |

| | | | | | | |
|---|---|---|---|---|---|---|
| IC50 values given in M; n.t., not tested | | | | | | |

The data in TABLE 1 show that especially the compounds PQ012 and PQ013 show a highly specific activity according to the invention.

## Claims

1. Use of a compound of general formula (I) 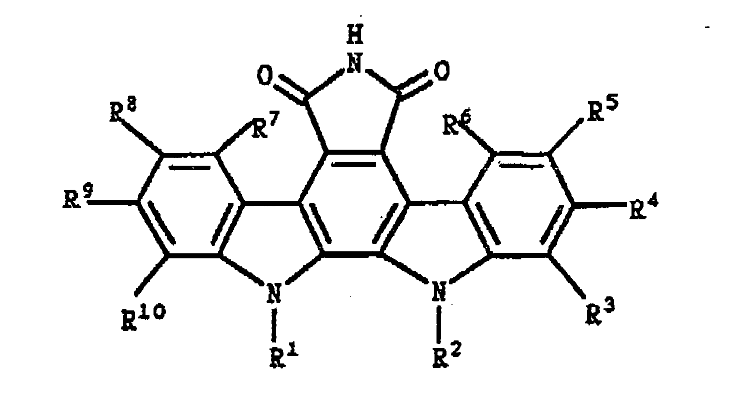 in which R¹ and R² are the same or different and, in each case, are a hydrogen atom or a methyl, ethyl, cyanomethyl, 2-cyanoethyl, 3-cyanopropyl, 3-cyano-2-propyl, 2-azidoethyl, 3-azidopropyl, 2,3-dihydroxypropyl, 3-chloro-2-hydroxy-1-propyl, 3-amino-propyl, 3-dimethylaminopropyl, 3-trimethylammoniopropyl, 4-aminobutyl, 5-aminopentyl, epoxymethyl, 3-methylaminopropyl, 3-ethylaminopropyl, 3-isopropylaminopropyl, 3-diisopropylaminopropyl, 3-methylemino-2 -hydroxy-1-propyl, 3 -ethylamino-2 - hydroxy-1-propyl, 3-isopropylamino-2-hydroxy-1-propyl, 3-dimethylamino-2-hydroxy-l-propyl, 3-diethylamino-2-hydroxy-1-propyl, 3-diisopropylamino-2-hydroxy-1-propyl, 3-pyrrolidino-2-hydroxy-1-propyl or the radical - (CH₂)₂CONH₂ and R³ to R¹⁰, independently of one another, each stand for hydrogen, methyl, methoxy, n-propoxy, chlorine, bromine, nitro, hydroxyl or amino or two neighbouring radicals together stand for a methylenedioxy radical, as well as of the pharmacologically acceptable salts thereof for preparing pharmaceutical compositions for the inhibition of the kinases FLT3 wild type mutant as FLT D835 or FLT3-ITD, and PDGF-Rα, PDGF-Rβ, CHK2, FAK, MAP4K4, Aurora B, PLK4, PRKG2,and RSK1-4 in humans as promoting factors for hematologic and solid tumors.

2. Use of a compound according to claim 2, wherein at least one of the radicals R¹ and R² is other than a hydrogen atom.

3. Use of a compound according to claims 1 to 2, wherein in the general formula
(I) R¹ and/or R² have the meaning of hydrogen, C1-C5 alkyl, amino-C1-C5 alkyl, mono- or bis- hydroxylated amino- C1-C5 alkyl, C1-C3 alkylamino- C1-C5 alkyl, mono or bis hydroxylated C1-C3 alkylamino-C1-C5 alkyl, carbonamido- C1-C4 alkyl, cyano- C1-C3 alkyl and R³ to R¹⁰ hydrogen and/or hydroxy or C1-C3-alkoxy-groups.

4. Use of a compound of formula according to claims 1 to 3, wherein R¹ and/or R² is hydrogen, methyl, dimethylaminoethyl, dimethylaminopropyl, 2-amidoethyl, 2-hydroxy-3-dimethylaminopropyl or 2-cyanoethyl and the remaining radicals R³ - R¹⁰ are as defined in claims 1 to 3, and preferably are hydrogen, hydroxy and/or methoxy.

5. Use of at least one compound of formula (I) according to claims 1 to 4, selected from the group consisting of 13-(2-cyanoethyl)-6,7,12,13-tetrahydro-3-methoxy-12-methyl-5,7 dioxo-5H-indolo[2,3-a] pyrrolo [3,4-c]-carbazole, 12-(3-dimethylaminopropyl)-6,7,12,13-tetrahydro-3,9-dihydroxy-12-methyl-5,7 dioxo-5H-indolo[2,3-a] pyrrolo [3,4-c]-carbazole, 6,7,12,13-tetrahydro-12-methyl-5,7 dioxo-5H-indolo [2,3-a] pyrrolo [3,4-c]-carbazole, 6,7,12,13-tetrahydro-3,9-dimethoxy-12-methyl-5,7 dioxo-5H-indolo [2,3-a] pyrrolo [3,4-c]-carbazole, 12-(2-carbamidoethyl)-6,7,12,13-tetrahydro-5,1 dioxo-5H-indolo[ 2,3-a] pyrrolo [3,4-c]-carbazole and (+-)-12-(3-dimethylamino-2-hydroxy-1-propyl)-6,7,12,13-tetrahydro-3,9-dihydroxy-13-methyl -5,1 dioxo-5H-indolo[ 2,3-a] pyrrolo [3,4-c]-carbazole

6. Use of 6,7,12,13-tetrahydro-3,9-dimethoxy-12-methyl-5,7 dioxo-5H-indolo[2,3-
a] pyrrolo [3,4-c]-carbazole according to claim 5.

7. Use of a compound of general formula (I) according to claims 1 to 6 which have a chiral centre in the radicals R¹ to R¹⁰ and are used as stereo-isomers or in the form of enantiomers.

8. Use of at least one compound according to formula (I) as defined in claims 1 to 7 for preparing a composition inhibiting one or more deregulating protein kinases of the group consisting of FLT3, FLT3 wild type mutant as FLT D835 or FLT3-ITD, PDGF-Rα, PDGF-Rβ for the treatment of hematologic tumors, preferably acute myelogenous leukemia (AML).

9. Use of at least one compound according to formula (I) as defined in claims 1 to 7 for preparing a composition inhibiting one or more deregulating protein kinases of the group consisting of CHK2, FAK, MAP4K4, Aurora B, PLK4, PRKG2,and RSK1-4 for the treatment of different solid tumors, preferably lung, breast or colon tumors.

10. Use of at least one compound as defined in claims 1 to 6 for treating hematologic or solid tumors in humans.
